# EUROPEAN PATENT APPLICATION

(11) **EP 4 647 064 A1**
(43) Date of publication of application: **12.11.2025**
(21) Application number: 25742074.5
(22) Date of filing: 14.01.2025
(51) Int. Cl.: A61K 8/60, A61Q 7/00, A23L 33/125, A61K 31/7048, A61P 17/14

(54) **COMPOSITION COMPRISING MELOSIDE FOR ALLEVIATING HAIR LOSS AND IMPROVING HAIR GROWTH**

(30) Priority: 18.01.2024 KR 20240008124
(71) Applicant: Morechem Co., Ltd., Seoul 06628 (KR)
(72) Inventor: PARK, Hyun Jun, Yongin-si, Gyeonggi-do, 16954 (KR); WANG, Da Hye, Yongin-si, Gyeonggi-do, 16954 (KR); SONG, Ji Hoon, Yongin-si, Gyeonggi-do, 16954 (KR); LEE, Ji Ean, Yongin-si, Gyeonggi-do, 16954 (KR); JOO, Il Woo, Yongin-si, Gyeonggi-do, 16954 (KR)
(74) Representative: Tiburzi, Andrea
(86) International application number: PCT/KR2025/000768
(87) International publication number: WO 2025/155058

(57) **Abstract**

The present invention relates to a composition comprising meloside for alleviating hair loss and improving hair growth and, more specifically, to a composition for alleviating hair loss and improving hair growth, the composition comprising, as an active ingredient, meloside that allows the growth/proliferation of dermal papilla cells and inhibits the expression/activity of the androgen receptor.

## Description

### [Technical Field]

The present invention relates to a composition comprising Meloside for relieving hair loss and improving hair growth, and more specifically, to a composition comprising Meloside for relieving hair loss and improving hair growth as an active ingredient that promotes the growth/proliferation of dermal papilla cells and inhibits the expression/activity of androgen receptors.

### [Background Art]

The hair growth cycle consists of the growth phase (anagen), the regression phase (catagen), and the resting phase (telogen), and is maintained through a cycle of hair growth and hair loss. Specifically, it may be divided into the growth phase, when hair grows, the regression phase, when growth stops and the hair follicle shrinks, and the resting phase, when the dermal papilla begins to be active or produces hair, causing existing hair to fall out.

The growth phase of hair lasts about 3 to 5 years for men and 4 to 6 years for women, while the regression phase lasts about 60 to 90 days and the resting phase lasts about 90 to 120 days, after which natural hair loss occurs.

Hair loss is classified into male pattern hair loss, female pattern hair loss, telogen effluvium, alopecia areata, and stress-induced hair loss, and is caused by various factors such as genetics, environment, psychology, and hormonal changes. Hair loss refers to the simultaneous occurrence of hair growth cessation and hair loss, and means that the percentage of hair that has stopped growing is increasing.

In particular, male pattern hair loss is known to be caused by hormones. The male hormone testosterone is converted into dihydrotestosterone (DHT) by 5-alpha-reductase, which acts directly on the hair follicles. DHT induces the hair follicles to enter the catagen phase, causing hair loss. Excessive increase in DHT causes hair to thin and fall out, and promotes thinning and hair loss in the crown area, exacerbating hair loss.

In addition, DHT produced by testosterone directly acts on the dermal papilla cells to increase the expression of androgen receptors. The androgen receptor is a nuclear translocation factor that binds to DHT, causing nuclear translocation, which increases the expression of hair loss-inducing factors such as dickkopf1, TNF-α, TGF-α, and IL-1, and induces the inhibition of Wnt signaling factors, leading to hair loss. Therefore, methods that regulate the expression of androgen receptors in dermal papilla cells are used to treat hair loss caused by these factors.

Under these technical circumstances, the inventors made efforts to develop a composition for relieving hair loss and improving hair growth, particularly a composition for preventing male pattern hair loss or improving hair growth. As a result, they discovered that a composition comprising Meloside as an active ingredient has the effect of promoting the growth/proliferation of dermal papilla cells and inhibiting the expression/activity of androgen receptors, and thus completed the present invention.

### [Prior Art Literature]

Korean Patent Publication No. 10-2011-0064989 (June 15, 2011)

### [Disclosure of Invention]

### [Technical Problem]

The main purpose of the present invention is to provide a composition comprising Meloside, an isomer thereof, a hydrate thereof, a solvate thereof, or a salt thereof as an active ingredient for relieving hair loss and improving hair growth.

Another purpose of the present invention is to provide medicines, health functional foods, and cosmetics manufactured using the composition.

### [Solution to Problem]

In order to achieve the objectives, the present invention provides Meloside represented by the following Formula 1, preferably Meloside A represented by the following Formula 2 and/or Meloside L represented by the following Formula 3, an isomer thereof, a hydrate thereof, a solvate thereof, or a salt thereof as an active ingredient.

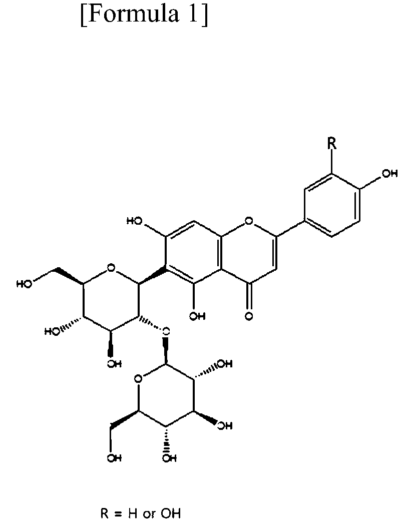

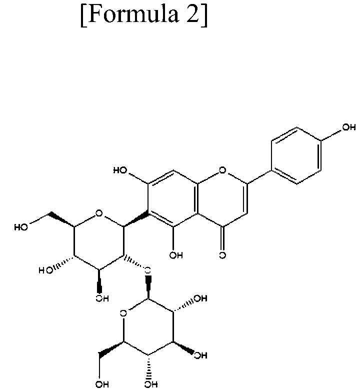

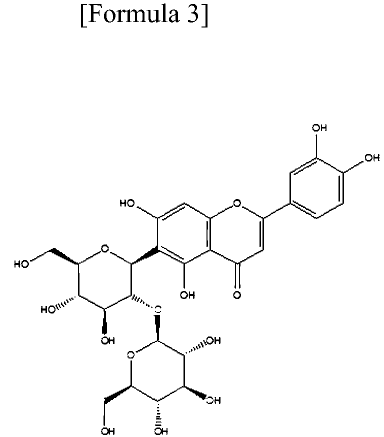

The present invention provides a composition for preventing male pattern hair loss or improving hair growth, which comprises Meloside represented by Formula 1, preferably Meloside A represented by Formula 2 and/or Meloside L represented by Formula 3, an isomer thereof, a hydrate thereof, a solvate thereof, or a salt thereof as the active ingredient.

In addition, the present invention provides a composition for promoting the growth/proliferation of dermal papilla cells and inhibiting the expression/activity of androgen receptors, which comprises Meloside represented by Formula 1, preferably Meloside A represented by Formula 2 and/or Meloside L represented by Formula 3, an isomer thereof, a hydrate thereof, a solvate thereof, or a salt thereof as the active ingredient.

In addition, the present invention provides medicines, health functional foods, and cosmetics manufactured using a composition comprising Meloside represented by Formula 1, preferably Meloside A represented by Formula 2 and/or Meloside L represented by Formula 3, an isomer thereof, a hydrate thereof, a solvate thereof, or a salt thereof as the active ingredient.

In addition, the present invention provides the use of Meloside represented by Formula 1, preferably Meloside A represented by Formula 2 and/or Meloside L represented by Formula 3, an isomer thereof, a hydrate thereof, a solvate thereof, or a salt thereof as the active ingredient in the manufacture of compositions for relieving hair loss and improving hair growth.

### [Advantageous Effects of Invention]

The composition comprising Meloside for relieving hair loss and improving hair growth according to the present invention is excellent in promoting the growth/proliferation of dermal papilla cells and inhibiting the expression/activity of androgen receptors, and can be used in the manufacture of cosmetics, medicines, etc. for preventing hair loss or improving hair growth.

### [Brief Description of Drawings]

FIG. 1 illustrates a graph of the growth/proliferation effects of Meloside A/Meloside L on dermal papilla cells according to an embodiment of the present invention.
FIG. 2 illustrates a graph of the growth/proliferation effects of Meloside A/Meloside L on outer root sheath cells according to an embodiment of the present invention.
FIG. 3 illustrates the improvement effect of Meloside A/Meloside L on male pattern hair loss according to an embodiment of the present invention, as indicated by Western blot results.
FIG. 4 illustrates the Western blot results of FIG. 3 quantified and represented as a graph.
FIG. 5 illustrates the androgen receptor nuclear translocation inhibitory effect of Meloside A/Meloside L according to an embodiment of the present invention, as observed by fluorescence microscopy.
FIG. 6 illustrates the fluorescence microscopy observation results of FIG. 5, quantified and presented as a graph.
FIG. 7 illustrates the graph representing the inhibitory effect of Meloside A/Meloside L on DKK1 expression according to an embodiment of the present invention.
FIG. 8 illustrates the graph representing the VEGF-increasing effect of Meloside A/Meloside L according to an embodiment of the present invention.
FIG. 9 illustrates a graph illustrating the inhibitory effect of Meloside A/Meloside L on cell death induced by dihydrotestosterone according to an embodiment of the present invention.
FIG. 10 illustrates a graph illustrating the inhibitory effect of Meloside A/Meloside L on cell death induced by DKK1 according to an embodiment of the present invention.
FIG. 11 illustrates a graph illustrating the hair loss inflammation relieving effect of Meloside A/Meloside L according to an embodiment of the present invention.
FIG. 12 illustrates a fluorescence microscope observation illustrating the active oxygen inhibitory effect of Meloside A/Meloside L according to an embodiment of the present invention.
FIG. 13 illustrates a graph quantifying the results of the fluorescence microscopy observations in FIG. 12.

### [Mode for the Invention]

Unless otherwise defined, all technical and scientific terms used in the present specification have the same meanings as those commonly understood by those skilled in the art to which the present invention pertains. In general, the nomenclature used in the present specification is well known and commonly used in the art.

Hereinafter, the present invention will be described in more detail.

In the present specification, the term "about" used to express length, area, volume, time (period), concentration, content, temperature, number, percentage (%), multiple, etc. means that there is a maximum tolerance of 10% in the corresponding numerical value or numerical range.

In one aspect, the present invention relates to a composition comprising Meloside represented by the following Formula 1, preferably Meloside A represented by the following Formula 2 and/or Meloside L represented by the following Formula 3, more preferably Meloside A, an isomer thereof, a hydrate thereof, a solvate thereof, or a salt thereof as an active ingredient, specifically compositions for relieving hair loss and improving hair growth.

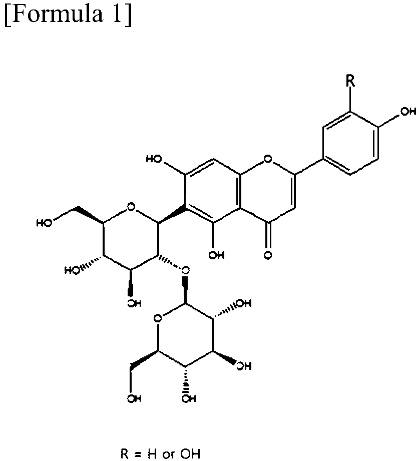

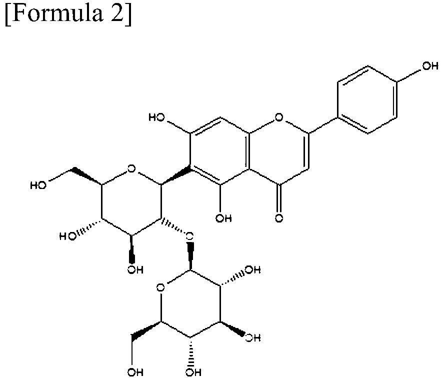

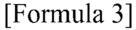

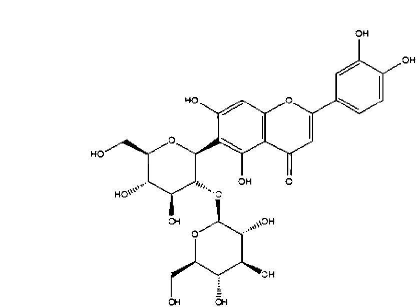

In addition, the present invention provides the use of Meloside represented by the Formula 1, preferably Meloside A represented by the Formula 2 and/or Meloside L represented by the Formula 3, an isomer thereof, a hydrate thereof, a solvate thereof, or a salt thereof as an active ingredient in the manufacture of compositions for relieving hair loss and improving hair growth.

Meloside A (isovitexin 2"-O-glucoside) is represented by Formula 2, and Meloside L (isoorientin 2"-O-glucoside) is represented by Formula 3 (see
https://pubchem.ncbi.nlm.nih.gov/compound/Meloside-A;and
https://pubchem.ncbi.nlm.nih.gov/compound/Meloside-L).

In the present specification, the term isomer refers to including not only stereoisomers (including optical isomers) but also conformation isomers (i.e., isomers that differ only in the angle of one or more chemical bonds), position isomers (particularly tautomers), or geometric isomers (e.g., cis-trans isomers).

Also, in the present specification, 'hydrate' refers to a compound in which water is bound, and includes inclusion compounds where there is no chemical bonding force between the water and the compound.

Also, in the present specification, "solvate" refers to a compound formed between the molecules or ions of a solute and the molecules or ions of a solvent.

In the present invention, the hair refers to any hair or fur on the body of a mammal, including humans, such as head hair (hair), eyebrows, eyelashes, mustaches, beards, chest hair, back hair, arm hair, leg hair, genital hair, nose hair, or ear hair, but is not limited thereto, and preferably refers to scalp hair (head hair).

In the present invention, the composition may be characterized by increasing the growth/proliferation of dermal papilla cells and decreasing the expression of androgen receptors.

In the present invention, the composition may be characterized by preventing the death of dermal papilla cells, alleviating scalp inflammation, and reducing the generation of reactive oxygen species, which are factors causing hair loss.

In the present invention, the content of the active ingredient included in the composition may be characterized by being 10 to 1000 ppm, preferably 10 to 100 ppm, and most preferably 20 to 30 ppm, based on the total weight of the composition, and if the content of Meloside, preferably one or more of Meloside A and Meloside L, is less than 10 ppm based on the total weight of the composition, the effect of relieving hair loss and improving hair growth is minimal, and if it exceeds 100 ppm, the increase in effect is not significant compared to the increase in content, and it may reduce formulation stability and usability.

The composition can be commercialized in various forms (cosmetics, health functional foods, medicines, etc.), and can be appropriately formulated considering the functionality, cost, and other conditions of the product to be implemented.

The composition may be a cosmetic composition, for example, a solution, suspension, emulsion, paste, gel, cream, lotion, powder, shampoo, conditioner, and spray, and the formulation is not particularly limited and can be appropriately selected according to the purpose.

The composition may be a health functional food composition, such as a health supplement, and may be in the form of a powder, granule, tablet, capsule, or beverage, but is not limited to these forms.

The active ingredient may be a prodrug of Meloside. A prodrug refers to a drug that has been chemically modified to adjust its physical and chemical properties, and that does not exhibit physiological activity on its own, but can be converted into the original drug through chemical or enzymatic action after administration, thereby exerting its pharmacological effects.

The composition is a pharmaceutical composition and may be in the form of, for example, tablets, ointments, lotions, gels, creams, sprays, suspensions, emulsions, patches, etc., but is not limited to these forms.

### [Mode for Invention]

Hereinafter, the present invention will be described in more detail by way of examples. These examples are provided solely for the purpose of illustrating the present invention, and it will be obvious to those skilled in the art that the scope of the present invention is not limited by these examples.

### [Reagent]

Meloside A was obtained from ChemFace, and Meloside L was separated/purified according to the method described below and applied to the experiment. Unless otherwise indicated, the reagents used in the cell culture and test examples 1 and 2 described below are products of Sigma-Aldrich.

### [Isolation and purification of Meloside L]

Meloside L was isolated and purified from china pink (*Dianthus chinensis*) and applied to experiments. The aerial parts of china pink were subjected to reflux extraction with 70% ethanol, and the resulting soft extract concentrated with a rotary vacuum evaporator was subjected to solvent fractionation using n-hexane, ethyl acetate, and n-butanol. The ethyl acetate fraction was applied to column chromatography to isolate a single compound, using silica gel and ODS (octadecylsilane) as stationary phases. The structure of the isolated/purified substance was confirmed through NMR (¹H, ¹³C NMR) analysis.

### [Cell Culture]

Human hair dermal papilla cells (HDPC), used in the test examples described below, were cultured in Dulbecco's Modified Eagle Medium (DMEM, WELGENE) supplemented with 10% Fetal Bovine Serum (FBS, WELGENE) and 1% antibiotics (Penicillin-Streptomycin, WELGENE) as the culture medium, in an incubator at 37°C, 5% CO₂, and humid conditions.

Human Hair Outer Root Sheath Cells (ORS), used in the test examples described below, were cultured in culture plates coated with Poly-L-Lysine, using Mesenchymal Stem Cell Medium (MSCM, ScienCell) supplemented with 5% Fetal Bovine Serum (FBS, ScienCell), 1% Mesenchymal stem cell growth supplement (MSCGS, ScienCell), and antibiotics (Penicillin-Streptomycin, ScienCell) as the culture medium, in an incubator at 37°C, 5% CO₂, and humid conditions.

### [Statistical processing]

Statistical analysis was performed using the t-test to test for significance between the two groups, and differences were considered significant when *P<0.05, **P<0.01, and ***P<0.001 compared to the negative control group.

### [Test Example 1] Confirmation of the cytotoxicity of Meloside on dermal papilla cells and outer root sheath cells

It was confirmed whether Meloside exhibited cytotoxicity towards dermal papilla cells and outer root sheath cells, thereby affecting their growth/proliferation.

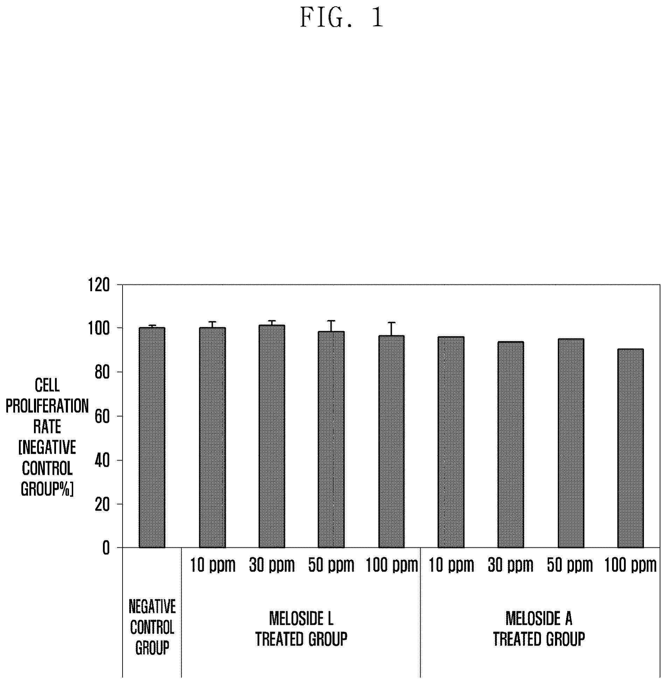

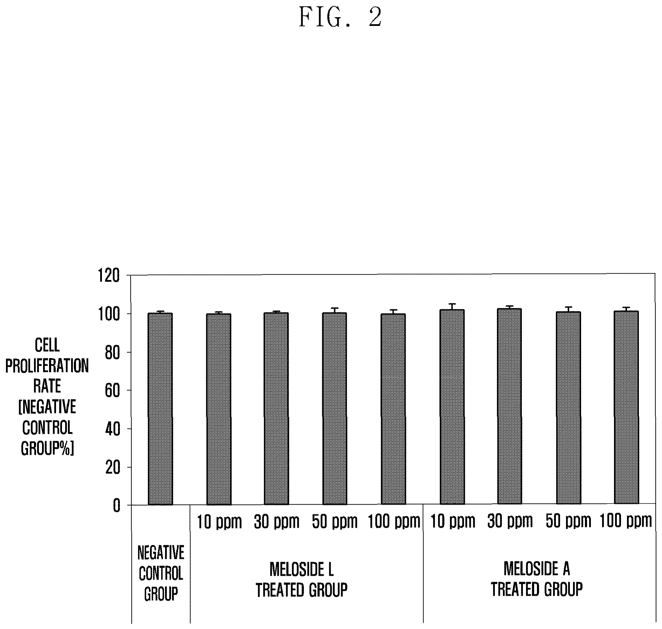

Specifically, after treating human dermal papilla cells (HDPC) or human outer root sheath cells (ORS) with Meloside A or Meloside L at concentrations of 10, 30, 50, and 100 ppm for 1 day, CCK-8 (Cell Counting Kit-8, Dojindo, EU) reagent was added to the treated HDPC according to the manufacturer's protocol. Absorbance was then measured at a wavelength of 450 nm using a microplate reader (BioTeK, USA), and the extent of HDPC growth/proliferation by Meloside treatment was compared and confirmed against the negative control group (treated with DMEM instead of Meloside A or L). The results for HDPC treatment are shown in FIG. 1, and the results for ORS treatment are shown in FIG. 2.

As shown in FIGS. 1 and 2, HDPC and ORS treated with Meloside A or L exhibited comparable levels of cell growth/proliferation to the negative control group. This confirmed that there was no effect such as cytotoxicity that would reduce the growth/proliferation of dermal papilla cells and outer root sheath cells.

### [Test Example 2] Confirmation of the androgenetic alopecia improvement effect by Meloside treatment

In this test example, the androgenetic alopecia improvement efficacy of Meloside (specifically, Meloside A and L) was confirmed by evaluating its effect on the expression of androgen receptors.

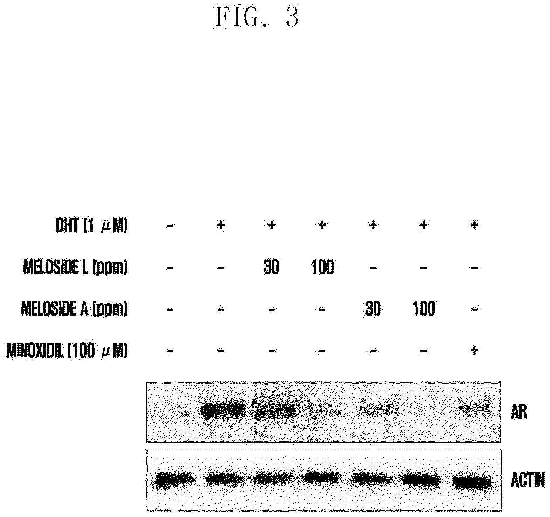

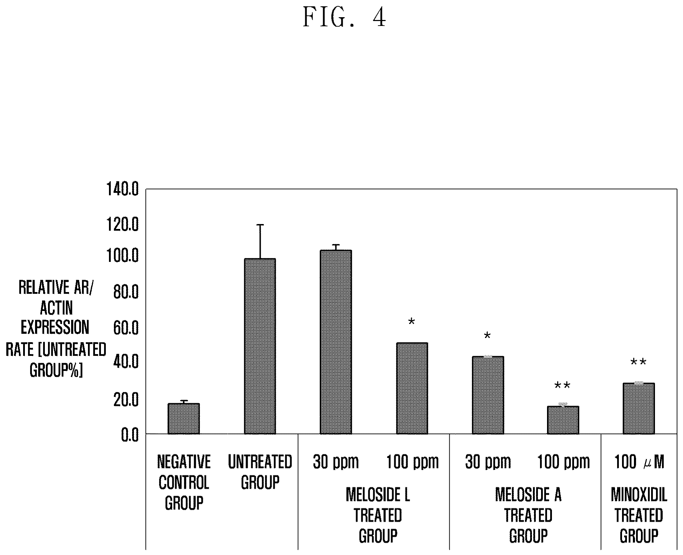

Specifically, the following groups were prepared: (i) a negative control group, where neither Dihydrotestosterone (DHT) nor Meloside A and L were treated on human dermal papilla cells (HDPC); (ii) an untreated group, where 1 µM DHT was treated on HDPC, but neither Meloside A nor L was treated; (iii) a positive control group, where 1 µM DHT was treated on HDPC, followed by treatment with 100 µM Minoxidil instead of Meloside A and L; and (iv) a treated group, where HDPC were first treated with 1 µM DHT to activate androgen receptors, and then treated with Meloside A or L at concentrations of 30 or 100 ppm, respectively, for 1 day. After preparation, the HDPC from (i) to (iv) were lysed, and the increase or decrease in the expression level of Androgen Receptor (AR) was confirmed using the Western blot technique. Here, for the analysis of relative expression patterns for each sample, the expression level was normalized based on the expression level of Actin. The expression patterns confirmed by Western blot are shown in FIG. 3, and the results of relative AR expression levels normalized based on Actin expression are shown in FIG. 4.

As shown in FIGS. 3 and 4, the decrease in androgen receptor (AR) expression in HDPC treated with Meloside A or L was concentration-dependent, reducing the formation of AR and DHT complexes. This confirmed a hair loss improvement effect (such as hair loss prevention) because of the reduction in AR activity.

In addition, it was confirmed that Meloside A had a superior effect in decreasing the expression level of androgen receptor (AR) compared to Meloside L.

### [Test Example 3] Confirmation of the inhibitory effect of androgen receptor nuclear translocation by Meloside treatment

Androgen receptor, as a transcription factor, is a protein that binds to specific regions of DNA to regulate transcription. Specific transcription factors play a role in mediating the activation or inhibition of particular genes.

It is known that the nuclear translocation of androgen receptors inhibits Wnt signaling pathway genes that inhibits hair loss, and increases the expression of DKK1, a hair loss-inducing protein (Cell Death & Disease volume 11, Article number: 407 (2020)).

To confirm the involvement of androgen receptors in hair loss-related gene transcription, recombinant plasmid EGFP-AR (Addgene) was used, and the extent of androgen receptor nuclear translocation in dermal papilla cells was measured using a cellular injection method.

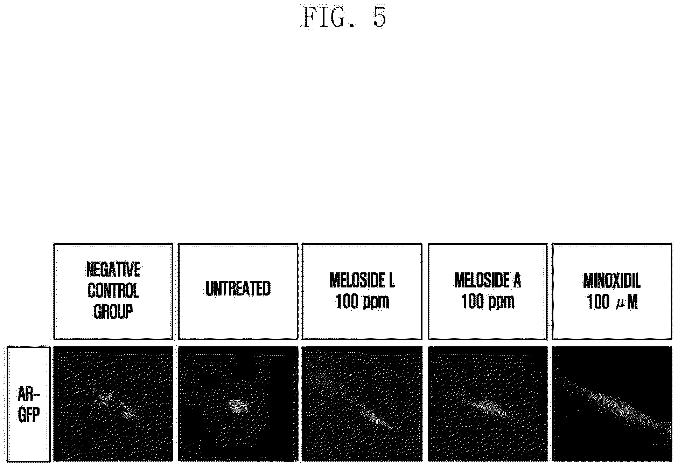

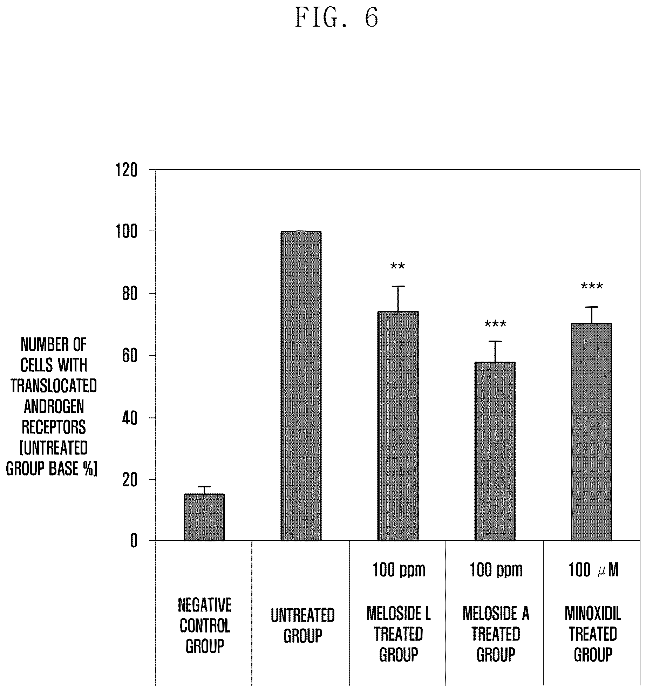

For the untreated group, only DHT was treated, and for the negative control group, neither DHT nor the sample was treated. In the experimental group, DHT and Meloside A or L at 100 ppm were co-treated, and after 24 hours, measurements were taken using a fluorescence microscope at Ex 485 nm and Em 535 nm. The results observed with the fluorescence microscope are shown in FIG. 5, and the number of cells with translocated androgen receptors in the treated groups, based on the untreated group, is shown in FIG. 6.

As shown in FIGS. 5 and 6, it was confirmed that the DHT-induced translocation of androgen receptors was inhibited by Meloside A and L. The effect of inhibiting androgen receptor translocation confirmed a hair loss relieving effect because of the reduction in the expression of hair loss-inducing genes. It was confirmed that the efficacy of Meloside A was superior to that of Meloside L treatment.

### [Test Example 4] Confirmation of the inhibitory effect of DKK1 expression by Meloside treatment

The efficacy of Meloside in regulating the expression level of DKK1, a downstream target of the androgen receptor, was confirmed.

HDPC were treated with DHT to increase DKK1 expression, and then treated with Meloside A or L at concentrations of 30 or 100 ppm, respectively, for 1 day to obtain the cell supernatant. For comparison, HDPC treated with Dihydrotestosterone (DHT) but without Meloside A and L were used as the untreated group, and HDPC treated with 100 µM Minoxidil were used as the positive control group.

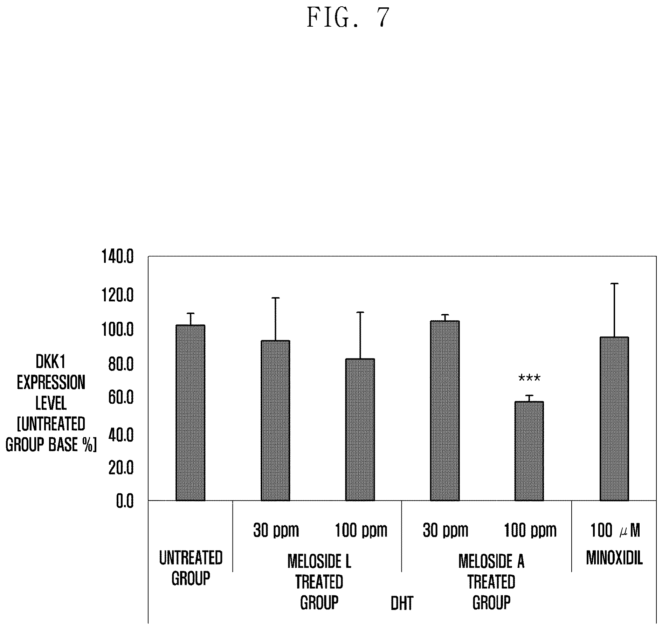

The increase or decrease in DKK1 expression level in the obtained cell supernatant was confirmed through an Enzyme-Linked Immunosorbent Assay (ELISA) method, and the results are shown in FIG. 7.

As shown in FIG. 7, the concentration-dependent decrease in DKK1 expression in HDPC treated with Meloside A was confirmed to relieve hair loss by reducing the inhibition of the Wnt signaling pathway. However, no effect was observed with Meloside L.

### [Test Example 5] Confirmation of the VEGF effect by Meloside treatment

To confirm the hair growth-promoting effect of Meloside A and L, an evaluation of the expression level of Vascular Endothelial Growth Factor (VEGF), a growth factor known to promote hair growth by being involved in vascular dilation and formation, was conducted.

HDPC were treated with Meloside A or L at concentrations of 30 or 100 ppm, respectively, for 1 day to obtain the cell supernatant. For comparison, HDPC that were not treated with either Meloside A or L were used as the negative control group, and HDPC treated with 100 µM Minoxidil were used as the positive control group.

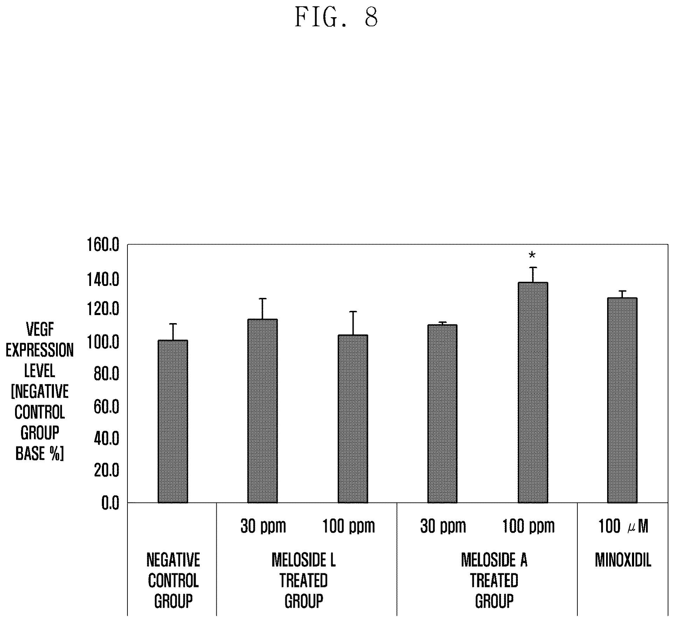

The increase or decrease in VEGF expression level in the obtained cell supernatant was confirmed through an Enzyme-Linked Immunosorbent Assay (ELISA) method, and the results are shown in FIG. 8.

As shown in FIG. 8, the increase in VEGF expression in HDPC treated with Meloside A and L was confirmed to aid in improving blood circulation, thereby demonstrating a hair loss relieving effect, and the blood circulation improvement effect of Meloside A was confirmed to be superior to that of Meloside L.

### [Test Example 6] Confirmation of the inhibitory effect of Dihydrotestosterone-induced cell death by Meloside treatment

The protective effect of Meloside A and L against DHT-induced death of dermal papilla cells (HDPC) was confirmed.

Specifically, HDPC were treated with Meloside A or L at concentrations of 30 or 100 ppm, respectively, for 1 day, and then cell death was induced with Dihydrotestosterone (DHT). For comparison, HDPC that were neither treated with Meloside A or L nor with DHT were used as the negative control group. HDPC treated with DHT but without Meloside A and L were used as the untreated group. HDPC treated with 100 µM Minoxidil or 5 mM NAC (N-acetyl cysteine) followed by DHT treatment were used as the positive control group.

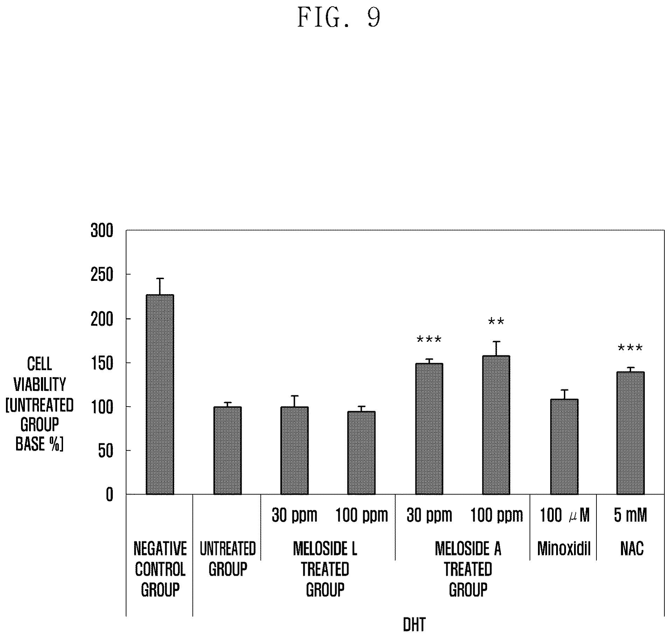

CCK-8 (Cell Counting Kit-8, Dojindo, EU) reagent was added to the HDPC according to the manufacturer's protocol, and absorbance was measured at a wavelength of 450 nm using a microplate reader (BioTeK, USA) to confirm the cell viability of dermal papilla cells because of Meloside treatment, compared to the negative control group (treated with DMEM instead of Meloside A or L). The results are shown in FIG. 9.

As shown in FIG. 9, a cell protective effect against DHT was confirmed in HDPC treated with Meloside A. Conversely, no cell protective effect against DHT was observed in HDPC treated with Meloside L.

### [Test Example 7] Confirmation of the inhibitory effect of DKK1-induced cell death by Meloside treatment

The protective effect of Meloside A and L against DKK1-induced death of dermal papilla cells (HDPC) was confirmed.

Specifically, HDPC were treated with Meloside A or L at concentrations of 30 or 100 ppm, respectively, for 1 day, and then cell death was induced with DKK1 (dickkopf WNT signaling pathway inhibitor 1). For comparison, HDPC that were neither treated with Meloside A or L nor with DHT were used as the negative control group. HDPC treated with DHT but without Meloside A and L were used as the untreated group. HDPC treated with 100 µM Minoxidil or 5 mM NAC followed by DHT treatment were used as the positive control group.

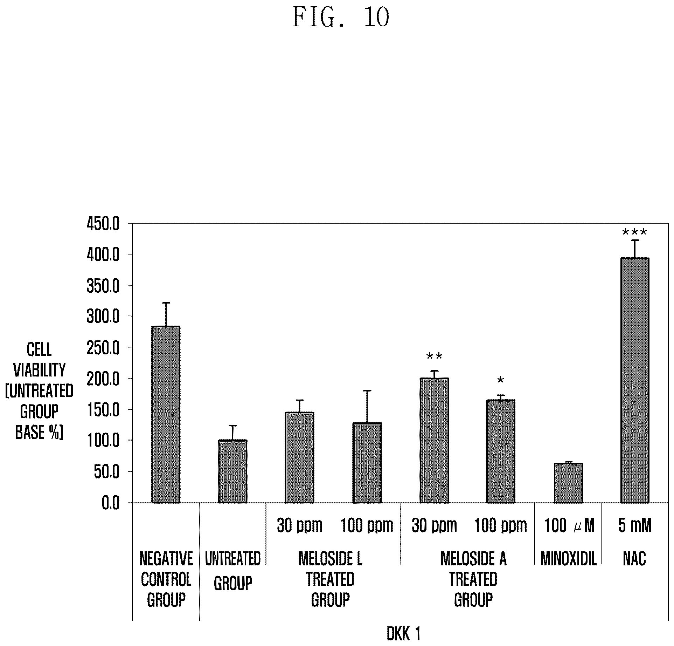

CCK-8 (Cell Counting Kit-8, Dojindo, EU) reagent was added to the HDPC according to the manufacturer's protocol, and absorbance was measured at a wavelength of 450 nm using a microplate reader (BioTeK, USA) to confirm the cell viability of dermal papilla cells because of Meloside treatment, compared to the negative control group (treated with DMEM instead of Meloside A or L). The results are shown in FIG. 10.

As shown in FIG. 10, a cell protective effect against DKK1 was confirmed in HDPC treated with Meloside A. Conversely, no cell protective effect against DKK1 was observed in HDPC treated with Meloside L.

### [Test Example 8] Confirmation of the relieving effect of hair loss inflammation by Meloside treatment

The inflammation-relieving efficacy of Meloside A and L was confirmed.

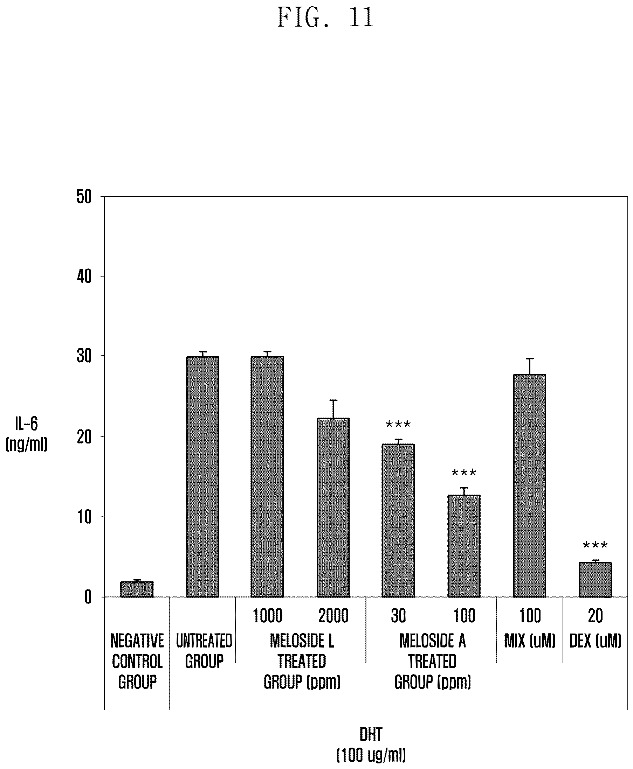

Specifically, the following groups were prepared: (i) a negative control group, where neither Dihydrotestosterone (DHT) nor Meloside A and L were treated on human dermal papilla cells (HDPC); (ii) an untreated group, where 100 ug/mL DHT was treated on HDPC, but neither Meloside A nor L was treated; (iii) a positive control group, where HDPC were treated with 100 µM Minoxidil or 20 µM Dexamethasone (DEX); and (iv) a treated group, where HDPC were first treated with 100 ug/mL DHT to increase IL-6 expression, and then treated with Meloside A or L at concentrations of 30 or 100 ppm, respectively, for 1 day. After preparation, the cell supernatants from HDPC of (i) to (iv) were obtained, and the increase or decrease in the expression level of IL-6 (Interleukin 6) was confirmed using an Enzyme-Linked Immunosorbent Assay (ELISA). For the analysis of expression patterns for each sample, normalization was performed based on a standard sample of IL-6, and the results are shown in FIG. 11.

As shown in FIG. 11, the decrease in IL-6 expression in HDPC treated with Meloside A was confirmed to reduce DHT-induced inflammation, thereby demonstrating an effect of relieving scalp inflammation because of reduced IL-6 expression. Conversely, no IL-6 expression reduction effect was observed in HDPC treated with Meloside L.

### [Test Example 9] Confirmation of the inhibitory effect of Dihydrotestosterone (DHT)-induced reactive oxygen species (ROS) by Meloside treatment

The intracellular reactive oxygen species inhibition effect was evaluated using human hair dermal papilla cells (HDPC). Measurement was performed by utilizing the principle that the non-fluorescent substance 2',7'-dichlorodihydrofluorescein diacetate (DCF-DA) reacts with intracellularly generated free radicals and oxidizes into the fluorescent substance 2',7'-dichlorofluorescein (DCF).

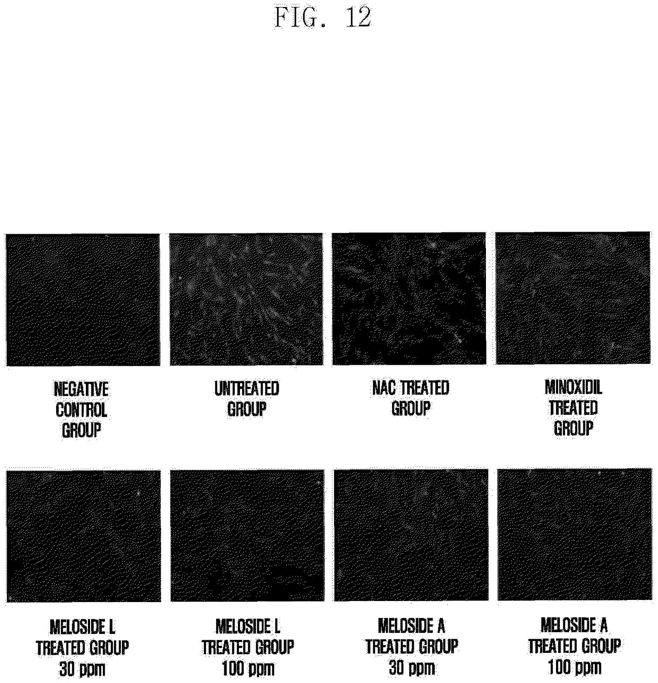

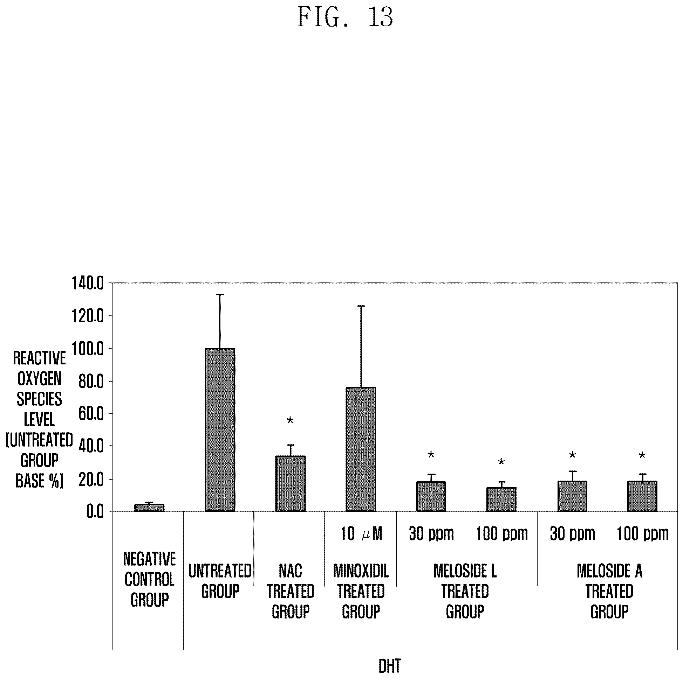

Human dermal papilla cells were seeded into 12-well plates at 5.5 x 10⁴ cells/well and cultured for 24 hours in a 37°C, 5% CO₂ incubator. Subsequently, samples prepared at various concentrations were treated into each well and cultured for 23 hours in a 37°C, 5% CO₂ incubator. For the treated groups, Meloside A or L at 30 or 100 ppm were treated along with DHT. For control groups, HDPC treated with 100 ug/mL DHT but without Meloside A and L, and untreated HDPC (neither Meloside A or L nor DHT) were used. Additionally, 5 mM NAC or 10 µM Minoxidil were used as positive control groups instead of Meloside A or L. After culturing the wells with 100 ug/mL DHT or Meloside for 24 hours in the incubator, 2',7'-dichlorodihydrofluorescein diacetate (DCF-DA, a fluorescent probe) was added and reacted for 30 minutes at room temperature. After removing DCF-DA and washing twice with PBS, images were taken every 10 minutes for 30 minutes using a fluorescence microscope at Ex 485 nm and Em 535 nm. The level of intracellular reactive oxygen species generation was measured by quantifying fluorescence intensity in 100 cells using Image J, thereby confirming the ROS reduction efficacy of Meloside. The results are shown in FIGS. 12 and 13.

As shown in FIGS. 12 and 13, the reduction in reactive oxygen species (ROS) generation in HDPC treated with Meloside A and L confirmed a hair loss improvement effect (such as hair loss prevention) by inhibiting DHT-induced ROS, a hair loss-inducing factor.

In view of the foregoing, specific parts of the present invention have been described in detail and it will be apparent to those skilled in the art that such specific descriptions are merely preferred embodiments and do not limit the scope of the present invention. Therefore, the true scope of the present invention is defined by the appended claims and their equivalents.

## Claims

1. A composition for relieving hair loss and improving hair growth, comprising: Meloside represented by the following Formula 1, an isomer thereof, a hydrate thereof, a solvate thereof, or a salt thereof as an active ingredient:

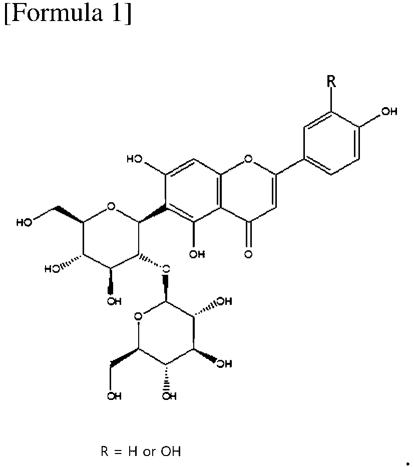

2. The composition of claim 1, wherein the Meloside is one or more selected from the group consisting of meloside A represented by the following Formula 2 and meloside L represented by the following Formula 3:

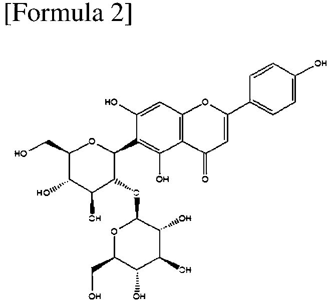

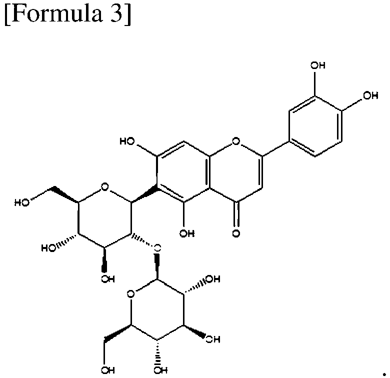

3. The composition of claim 1 or claim 2, wherein the composition is for preventing or improving male pattern hair loss.

4. The composition of claim 1 or claim 2, wherein the composition is for increasing the growth of the dermal papilla cell.

5. The composition of claim 1 or claim 2, wherein the composition is for reducing the expression of the androgen receptor.

6. The composition of claim 1 or claim 2, wherein the composition is for preventing the death of dermal papilla cell.

7. The composition of claim 1 or claim 2, wherein the composition is for relieving the scalp inflammation.

8. The composition of claim 1 or claim 2, wherein the composition is for reducing the generation of reactive oxygen species (ROS), which is a hair loss-inducing factor.

9. The composition of claim 1 or claim 2, wherein the composition is cosmetic composition, health functional food composition, or pharmaceutical composition.

10. Meloside represented by Formula 1, an isomer thereof, a hydrate thereof, a solvate thereof, or a salt thereof as an active ingredient for use in the treatment of hair loss:

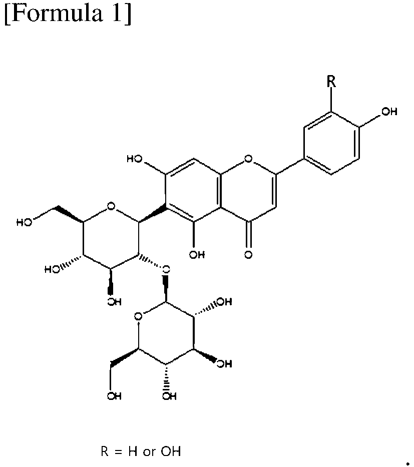

11. Meloside according to claim 10, for use according to claim 10, wherein the Meloside is one or more selected from the group consisting of Meloside A represented by the following Formula 2 and Meloside L represented by the following Formula 3:

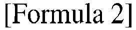

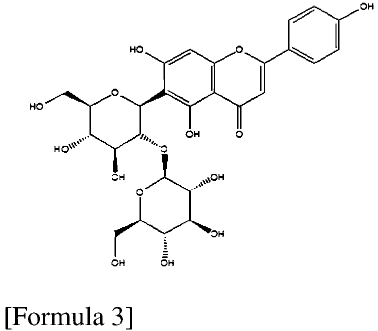

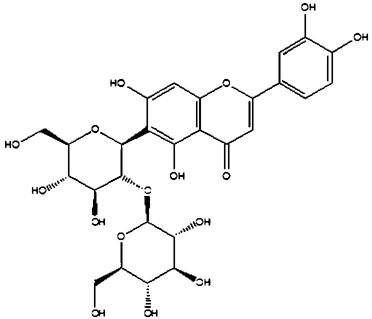

12. Cosmetic use of Meloside represented by Formula 1, an isomer thereof, a hydrate thereof, a solvate thereof, or a salt thereof as an active ingredient for relieving hair loss and improving hair growth:

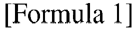

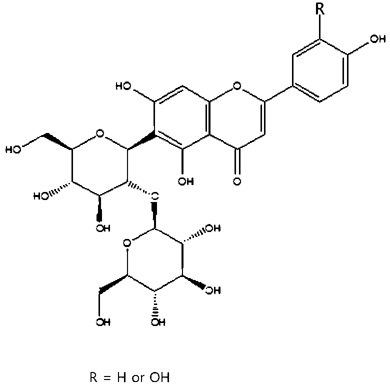

13. Cosmetic use according to claim 12, wherein the Meloside is one or more selected from the group consisting of Meloside A represented by the following Formula 2 and Meloside L represented by the following Formula 3:
